# EUROPEAN PATENT SPECIFICATION

(11) **EP 3 193 819 B1**
(45) Date of publication and mention of the grant of the patent: **08.01.2020**
(21) Application number: 14904141.0
(22) Date of filing: 15.10.2014
(51) Int. Cl.: A61K 8/27, A61K 8/44, A61Q 11/00, A61K 8/365

(54) **ORAL CARE COMPOSITIONS COMPRISING ZINC, ARGININE AND SERINE**
MUNDPFLEGEZUSAMMENSETZUNGEN MIT ZINK, ARGININ UND SERIN
COMPOSITIONS DE SOINS BUCCAUX COMPRENANT DU ZINC, DE L'ARGININE ET DE LA SÉRINE

(43) Date of publication of application: 26.07.2017
(73) Proprietor: Colgate-Palmolive Company, New York, NY 10022 (US)
(72) Inventor: XU, Shao Peng, Guangzhou 510730 Guangdong (CN); HASSAN, Mahmoud, Somerset, New Jersey 08873 (US); HUANG, Xiaoyi, Guangzhou Guangdong 510623 (CN)
(74) Representative: Wibbelmann, Jobst
(86) International application number: PCT/CN2014/088622
(87) International publication number: WO 2016/058140

(56) References cited:
- WO-A1-2011/053273
- WO-A1-2011/123123
- WO-A1-2011/162756
- WO-A1-2014/088572
- WO-A1-2014/088575

## Description

### BACKGROUND

Dental plaque is a biofilm that adheres to tooth and other oral surfaces, particularly at the gingival margin, and is implicated in the occurrence of gingivitis, periodontitis, caries and other forms of periodontal disease. Dental plaque is cohesive and highly resistant to removal from teeth and/or oral surfaces. Dental plaque comprises glucans, which are insoluble polysaccharides that provide plaque with its cohesive properties. The bacterial enzyme glucosyltransferase converts dietary sugar into glucans. Plaque mineralizes to form a hard deposit called calculus (or tartar), which becomes a local irritant for the gums, causing gingivitis.

Various antibacterial agents can retard the growth of bacteria and thus reduce the formation of biofilm on oral surfaces.

Zinc and other metal compounds/salts have been previously used as antibacterial agents. Without being bound by any theory, free zinc ions are believed to provide antibacterial efficacy by inhibition of glucose metabolism and/or interaction with the bacterial cell wall, reducing bacterial colonization of the oral cavity (as discussed in Cummins D., J Clin Periodontal 1991; 18; 455-461). An insoluble zinc compound, zinc oxide, could also deliver strong antibacterial efficacy during tooth brushing. Document WO 2014/088575 discloses oral care compositions comprising basic amino acid as well as zinc ions having an enhanced anti-bacterial efficacy, for ex. zinc oxide, zinc citrate and arginine. Document WO 2011/123123 discloses oral care compositions comprising metal oxide particles and amino acids capable of chelating the metal oxide, for ex. zinc oxide and arginine or zinc oxide and serine, for enhancing the delivery of the metal oxide.

It would be desirable to provide an oral care composition which exhibits even greater biofilm reduction efficacy than previously-known compositions.

### BRIEF SUMMARY

In a first aspect, the present invention provides an oral care composition comprising: (a) arginine, in free or salt form; (b) serine; (c) zinc oxide; and (d) zinc citrate.

Optionally, the total concentration of zinc oxide and zinc citrate in the composition is from 0.2 weight % to 5 weight %, based on the total weight of the composition.

Optionally, the weight ratio of zinc oxide to zinc citrate is from 1.5:1 to 4.5:1. Still further optionally, the weight ratio of zinc oxide to zinc citrate is from 1.5:1 to 4:1, from 1.7:1 to 2.3:1, from 1.9:1 to 2.1:1, or about 2:1.

Optionally, the composition comprises zinc oxide in an amount of from 0.5 weight % to 1.5 weight % and zinc citrate in an amount of from 0.25 weight % to 0.75 weight %, based on the total weight of the composition. Further optionally, the composition comprises zinc oxide in an amount of about 1 weight % and zinc citrate in an amount of about 0.5 weight %, based on the total weight of the composition.

Optionally, the arginine is present in an amount of from 0.5 to 8 weight % and the serine is present in an amount of from 0.01 to 0.8 weight %, based on the total weight of the composition. Further optionally, the arginine is present in an amount of from 0.5 weight % to 1.5 weight % and the serine is present in an amount of from 0.05 weight % to 0.2 weight %, based on the total weight of the composition.

Optionally, the weight ratio of serine to arginine is from 1:5 to 1:15, from 1:7 to 1:12, or about 1:10.

Optionally, the arginine is L-arginine.

Optionally, the serine is L-serine.

Optionally, the arginine is present as free arginine.

Optionally, the arginine is present as arginine hydrochloride, arginine bicarbonate, or arginine phosphate.

Optionally, the weight ratio of the zinc oxide and zinc citrate to the arginine and serine in the composition is from 2:1 to 1:1. Further optionally, the weight ratio of the zinc oxide and zinc citrate to the arginine and serine in the composition is about 1.4:1.

Optionally, the composition further comprises an abrasive. Further optionally, the abrasive is selected from a silica abrasive, aluminum oxide, aluminum silicate, calcined alumina, bentonite, insoluble phosphate, natural calcium carbonate, precipitated calcium carbonate, and mixtures thereof. Optionally, the abrasive is a silica abrasive. Optionally, the abrasive is natural calcium carbonate or precipitated calcium carbonate.

Optionally, the oral care composition is a dentifrice, a toothpaste, a gel, a tooth powder, a mouthwash, a mouthrinse, a lozenge, a tablet, a spray, a gum, or a film.

In a second aspect, the present invention provides an oral care composition comprising: (a) arginine, in free or salt form; (b) serine; (c) zinc oxide; and (d) zinc citrate, for use in reducing or inhibiting biofilm formation in an oral cavity.

Optionally, the total concentration of zinc oxide and zinc citrate in the composition is from 0.2 weight % to 5 weight %, based on the total weight of the composition.

Optionally, the weight ratio of zinc oxide to zinc citrate is from 1.5:1 to 4.5:1. Still further optionally, the weight ratio of zinc oxide to zinc citrate is from 1.5:1 to 4:1, from 1.7:1 to 2.3:1, from 1.9:1 to 2.1:1, or about 2:1.

Optionally, the composition comprises zinc oxide in an amount of from 0.5 weight % to 1.5 weight % and zinc citrate in an amount of from 0.25 weight % to 0.75 weight %, based on the total weight of the composition. Further optionally, the composition comprises zinc oxide in an amount of about 1 weight % and zinc citrate in an amount of about 0.5 weight %, based on the total weight of the composition.

Optionally, the arginine is present in an amount of from 0.5 to 8 weight % and the serine is present in an amount of from 0.01 to 0.8 weight %, based on the total weight of the composition. Further optionally, the arginine is present in an amount of from 0.5 weight % to 1.5 weight % and the serine is present in an amount of from 0.05 weight % to 0.2 weight %, based on the total weight of the composition.

Optionally, the weight ratio of serine to arginine is from 1:5 to 1:15, from 1:7 to 1:12, or about 1:10.

Optionally, the arginine is L-arginine.

Optionally, the serine is L-serine.

Optionally, the arginine is present as free arginine.

Optionally, the arginine is present as arginine hydrochloride, arginine bicarbonate, or arginine phosphate.

Optionally, the weight ratio of the zinc oxide and zinc citrate to the arginine and serine in the composition is from 2:1 to 1:1. Further optionally, the weight ratio of the zinc oxide and zinc citrate to the arginine and serine in the composition is about 1.4:1.

Optionally, the composition further comprises an abrasive. Further optionally, the abrasive is selected from a silica abrasive, aluminum oxide, aluminum silicate, calcined alumina, bentonite, insoluble phosphate, natural calcium carbonate, precipitated calcium carbonate, and mixtures thereof. Optionally, the abrasive is a silica abrasive. Optionally, the abrasive is natural calcium carbonate or precipitated calcium carbonate.

Optionally, the oral care composition is a dentifrice, a toothpaste, a gel, a tooth powder, a mouthwash, a mouthrinse, a lozenge, a tablet, a spray, a gum, or a film.

In a third aspect, the present invention provides a method of reducing or inhibiting biofilm formation in an oral cavity, the method comprising contacting the oral cavity with an oral care composition comprising: (a) arginine, in free or salt form; (b) serine; (c) zinc oxide; and (d) zinc citrate.

Optionally, the total concentration of zinc oxide and zinc citrate in the composition is from 0.2 weight % to 5 weight %, based on the total weight of the composition.

Optionally, the weight ratio of zinc oxide to zinc citrate in the composition is from 1.5:1 to 4.5:1. Still further optionally, the weight ratio of zinc oxide to zinc citrate in the composition is from 1.5:1 to 4:1, from 1.7:1 to 2.3:1, from 1.9:1 to 2.1:1, or about 2:1.

Optionally, the composition comprises zinc oxide in an amount of from 0.5 weight % to 1.5 weight % and zinc citrate in an amount of from 0.25 weight % to 0.75 weight %, based on the total weight of the composition. Further optionally, the composition comprises zinc oxide in an amount of about 1 weight % and zinc citrate in an amount of about 0.5 weight %, based on the total weight of the composition.

Optionally, the arginine is present in the composition in an amount of from 0.5 weight % to 8 weight % and the serine is present in the composition in an amount of from 0.01 weight % to 0.8 weight %, based on the total weight of the composition. Further optionally, the arginine is present in the composition in an amount of from 0.5 weight % to 1.5 weight % and the serine is present in the composition in an amount of from 0.05 weight % to 0.2 weight %, based on the total weight of the composition.

Optionally, the weight ratio of serine to arginine in the composition is from 1:5 to 1:15, from 1:7 to 1:12, or about 1:10.

Optionally, the arginine is L-arginine.

Optionally, the serine is L-serine.

Optionally, the arginine is present in the composition as free arginine.

Optionally, the arginine is present in the composition as arginine hydrochloride, arginine bicarbonate, or arginine phosphate.

Optionally, the weight ratio of the zinc oxide and zinc citrate to the arginine and serine in the composition is from 2:1 to 1:1. Further optionally, the weight ratio of the zinc oxide and zinc citrate to the arginine and serine in the composition is about 1.4:1.

Optionally, the composition further comprises an abrasive. Further optionally, the abrasive is selected from a silica abrasive, aluminum oxide, aluminum silicate, calcined alumina, bentonite, insoluble phosphate, natural calcium carbonate, precipitated calcium carbonate, and mixtures thereof. Optionally, the abrasive is a silica abrasive. Optionally, the abrasive is natural calcium carbonate or precipitated calcium carbonate.

Optionally, the oral care composition is a dentifrice, a toothpaste, a gel, a tooth powder, a mouthwash, a mouthrinse, a lozenge, a tablet, a spray, a gum, or a film.

In a fourth aspect, the present invention provides the use, in an oral care composition, of a combination of (a) arginine, in free or salt form, (b) serine, (c) zinc oxide, and (d) zinc citrate, to reduce or inhibit biofilm formation in an oral cavity.

Further areas of applicability of the present invention will become apparent from the detailed description provided hereinafter.

### DETAILED DESCRIPTION

The following description of the preferred embodiment(s) is merely exemplary in nature.

As used throughout, ranges are used as shorthand for describing each and every value that is within the range. Any value within the range can be selected as the terminus of the range.

Unless otherwise specified, all percentages and amounts expressed herein and elsewhere in the specification should be understood to refer to percentages by weight. The amounts given are based on the active weight of the material.

Unless otherwise specified, all ratios as expressed herein should be understood to refer to ratios by weight.

The present inventors have surprisingly found that the inclusion of a combination of arginine and serine in an oral care composition comprising the combination of zinc oxide and zinc citrate improves the efficacy of the oral care composition in reducing biofilm. The present inventors have also surprisingly found that zinc oxide and zinc citrate containing compositions which comprise both arginine and serine exhibit increased biofilm reduction efficacy as compared to zinc oxide and zinc citrate containing compositions which include serine but no arginine, and as compared to zinc oxide and zinc citrate containing compositions which include arginine but no serine.
In a first aspect, the present invention provides an oral care composition comprising: (a) arginine, in free or salt form; (b) serine; (c) zinc oxide; and (d) zinc citrate.
In a second aspect, the present invention provides an oral care composition comprising: (a) arginine, in free or salt form; (b) serine; (c) zinc oxide; and (d) zinc citrate, for use in reducing or inhibiting biofilm formation in an oral cavity.
In a third aspect, the present invention provides a method of reducing or inhibiting biofilm formation in an oral cavity, the method comprising contacting the oral cavity with an oral care composition comprising: (a) arginine, in free or salt form; (b) serine; (c) zinc oxide; and (d) zinc citrate.
In a fourth aspect, the present invention provides the use, in an oral care composition, of a combination of (a) arginine, in free or salt form, (b) serine, (c) zinc oxide, and (d) zinc citrate, to reduce or inhibit biofilm formation in an oral cavity.

In each of the above aspects, the total concentration of the zinc oxide and zinc citrate in the composition may be from 0.2 weight % to 5 weight %, from 0.5 to 2.5 weight %, from 1 to 2 weight %, or about 1.5 weight %, based on the total weight of the composition.

In any embodiments of each of the above aspects, the weight ratio of zinc oxide to zinc citrate may be from 1.5:1 to 4.5:1, from 1.5:1 to 4:1, from 1.7:1 to 2.3:1, from 1.9:1 to 2.1:1, or about 2:1.

In any embodiments of each of the above aspects, the oral care composition may comprise zinc oxide in an amount of from 0.5 weight % to 1.5 weight % and zinc citrate in an amount of from 0.25 weight % to 0.75 weight %, based on the total weight of the composition; or zinc oxide in an amount of from 0.75 weight % to 1.25 weight % and zinc citrate in an amount of from 0.4 weight % to 0.6 weight %, based on the total weight of the composition; or zinc oxide in an amount of about 1 weight % and zinc citrate in an amount of about 0.5 weight %, based on the total weight of the composition.

In any embodiments of each of the above aspects, the zinc citrate may be zinc citrate trihydrate.

In some embodiments of each of the above aspects, the arginine is present in the composition in an amount of from 0.5 weight % to 8 weight % and the serine is present in the composition in an amount of from 0.01 weight % to 0.8 weight %, based on the total weight of the composition; or the arginine is present in the composition in an amount of from 0.5 weight % to 6 weight % and the serine is present in the composition in an amount of from 0.01 weight % to 0.5 weight %, based on the total weight of the composition; or the arginine is present in the composition in an amount of from 0.5 weight % to 3 weight % and the serine is present in the composition in an amount of from 0.01 weight % to 0.25 weight %, based on the total weight of the composition; or the arginine is present in the composition in an amount of from 0.5 weight % to 1.5 weight % and the serine is present in the composition in an amount of from 0.05 weight % to 0.2 weight %, based on the total weight of the composition; or the arginine is present in the composition in an amount of from 0.75 weight % to 1.25 weight % and the serine is present in the composition in an amount of from 0.075 weight % to 0.15 weight %, based on the total weight of the composition; or the arginine is present in the composition in an amount of about 1 weight % and the serine is present in the composition in an amount of about 0.1 weight %, based on the total weight of the composition.

In any embodiments of each of the above aspects, the weight ratio of serine to arginine may be from 1:5 to 1:15, from 1:7 to 1:12, or about 1:10.

In any embodiments of each of the above aspects, the arginine may be L-arginine.

In any embodiments of each of the above aspects, the serine may be L-serine.

In any embodiments of each of the above aspects, the arginine may be present as free arginine. Alternatively, the arginine may be present as arginine hydrochloride, arginine bicarbonate, or arginine phosphate.

In each of the above aspects, the serine is present as free serine.

In any embodiments of each of the above aspects, the weight ratio of the zinc oxide and zinc citrate to the arginine and serine in the composition may be from 2:1 to 1:1, from 1.75:1 to 1.25:1, or about 1.4:1.

In any embodiments of each of the above aspects of the present invention, the composition may further comprise an abrasive. Suitable abrasives which may be included in the compositions of the present invention include, but are not limited to: silica abrasives, aluminum oxide, aluminum silicate, calcined alumina, bentonite, insoluble phosphates, natural calcium carbonate, precipitated calcium carbonate, and mixtures thereof. In some embodiments, the abrasive is a silica abrasive. Examples of silica abrasives include, but are not limited to, precipitated or hydrated silicas having a mean particle size of up to about 20 microns (such as Zeodent 105 and Zeodent 114 marketed by J.M. Huber Chemicals Division, Havre de Grace, Md. 21078); Sylodent 783 (marketed by Davison Chemical Division of W.R. Grace & Company); or Sorbosil AC 43 (from PQ Corporation). In some embodiments, the abrasive is a calcium carbonate abrasive, for example natural calcium carbonate or precipitated calcium carbonate.

Alternatively, the composition may be free from abrasives. By "free from abrasives", it is meant that the composition comprises less than 1 weight %, less than 0.5 weight %, less than 0.25 weight %, or 0 weight % abrasives, based on the total weight of the composition.

In any embodiments of each of the above aspects of the present invention, the oral care composition may be a dentifrice, a toothpaste, a gel, a tooth powder, a mouthwash, a mouthrinse, a lozenge, a tablet, a spray, a gum, or a film. In some embodiments, the oral care composition is a toothpaste or a gel. In other embodiments, the oral care composition is a mouthwash or a mouthrinse. In those embodiments where the composition is a mouthwash or a mouthrinse, the composition may be free from abrasives. In certain embodiments where the composition is a mouthwash or a mouthrinse, the composition may contain water in an amount of from 60 to 95 weight %, based on the weight of the total composition.

In any embodiments of each of the above aspects, the oral care compositions may further comprise additional ingredients. These additional ingredients may include, but are not limited to, diluents (e.g. water), bicarbonate salts, pH modifying agents, surfactants (such as anionic, nonionic or amphoteric surfactants), foam modulators, thickening agents, humectants, sweeteners, flavorants, pigments, additional antibacterial agents, anticalculus agents, anticaries agents, preservatives, and mixtures thereof.

The oral care compositions may also comprise at least one surfactant. Any orally acceptable surfactants, which may be anionic (e.g. sodium lauryl sulfate - SLS), nonionic or amphoteric (e.g. betaine), can be used. One or more surfactants are optionally present in a total amount of 0.01 weight % to 10 weight %, for example, from 0.05 weight % to 5 weight %, or from 0.1 weight % to 3.5 weight % by total weight of the oral care composition.

The oral care compositions may comprise at least one foam modulator, useful for example to increase amount, thickness or stability of foam generated by the composition upon agitation. One or more foam modulators are optionally present in a total amount of 0.1 weight % to 10 weight %, for example from 0.2 weight % to 5 weight %, or from 0.25 weight % to 2 weight %, by total weight of the oral care composition.

The oral care compositions may comprise at least one sweetener, useful for example to enhance taste of the composition. One or more sweeteners are optionally present in a total amount depending strongly on the particular sweetener(s) selected, but typically 0.005 weight % to 5 weight %, by total weight of the composition, optionally 0.01 weight % to 1 weight %, further optionally 0.1 weight % to 0.5 weight % by total weight of the oral care composition.

The oral care compositions may also comprise at least one flavorant, useful for example to enhance taste of the composition. One or more flavorants are optionally present in a total amount of from 0.01 weight % to 5 weight %, for example, from 0.03 weight % to 2.5 weight %, optionally 0.05 weight % to 1.5 weight %, further optionally 0.1 weight % to 0.3 weight % by total weight of the oral care composition.

The oral care compositions may comprise at least one colorant. Colorants herein include pigments, dyes, lakes and agents imparting a particular luster or reflectivity such as pearling agents. Any orally acceptable colorant can be used. One or more colorants are optionally present in a total amount of from 0.0001 weight % to 5 weight %, for example, from 0.0001 weight % to 1 weight %, or from 0.0005 weight % to 0.5 weight %, by total weight of the oral care composition.

The oral care compositions may also comprise a fluoride ion source. Fluoride ion sources may be added to the oral care compositions at a level of 0.001 weight % to 10 weight %, e.g., from 0.003 weight % to 5 weight %, from 0.01 weight % to 2 weight %, or 0.1 weight % to 1.5 weight %. However, it is to be understood that the weights of fluoride salts to provide the appropriate level of fluoride ion will obviously vary based on the weight of the counter ion in the salt, and one of skill in the art may readily determine such amounts.

The oral care compositions may comprise a saliva stimulating agent useful, for example, in amelioration of dry mouth. One or more saliva stimulating agents are optionally present in saliva stimulating effective total amount.

The oral care compositions may include antisensitivity agents. Such agents may be added in effective amounts, e.g., from 1 weight % to 20 weight % by weight based on the total weight of the oral care composition, depending on the agent chosen.

The oral care composition may include anticalculus agents. Such agents may be added to the oral care compositions at a level of from 0.1 weight % to 3 weight %, from 0.25 weight % to 1.5 weight %, from 0.4 weight % to 1 weight %, or about 0.5 weight %.

The oral care composition of the invention may further comprise an antioxidant.

### EXAMPLES

Experiments were carried out in order to evaluate the biofilm reduction efficacy of compositions containing a combination of zinc oxide and zinc citrate and a combination of arginine and serine, as compared to the biofilm reduction efficacy of (i) compositions containing a combination of zinc oxide (ZnO) and zinc citrate (ZnCit) but neither arginine nor serine; (ii) compositions containing a combination of zinc oxide, zinc citrate and arginine, but no serine; (iii) compositions containing a combination of zinc oxide, zinc citrate and serine, but no arginine; and (iv) compositions containing no zinc compounds and no amino acids.

In all of the Examples, below, the experimental methodology used to evaluate the biofilm growth inhibition of the compositions was as follows:
(1) Dental plaque was collected from four healthy volunteers and pooled together as inoculum. The Optical Density of the inoculum was matched to 0.3 absorbance at 610nm.
(2) Sterile hydroxyapatite (HAP) disks were incubated under anaerobic conditions at 37°C for 24 hours with 1mL of sterile artificial saliva (with 0.01 weight% sucrose) and 1mL of pooled saliva in a 24 well microplate.
(3) For each composition tested, a treatment solution of 1 part dentifrice: 2 parts sterile distilled water by weight was made up. Each freshly prepared treatment solution was added to three wells and allowed to contact the HAP disk therein for 10 minutes.
(4) The liquid phase of each well was then removed and was replaced by 2mL sterile artificial saliva.
(5) The disks were then maintained at 37°C under anaerobic conditions for 8 days.
(6) At intervals of 2, 4 and 8 days, the disks were collected aseptically and transferred to half-strength pre-reduced thioglycollate medium (4.5 mL per disk)
(7) 100µL of the dilution 10-4, 10-5 and 10-6 were plated in duplicates for each disk on Neomycin/Vancomycin (NV) Agar for Total Gram-negative Anaerobes.
(8) The plates were surface-spread using a sterile spreader and were incubated anaerobically at 37°C for 72 hours, after which time the number of colonies on each plate was counted.

The log10 CFU/ml (where CFU = colony forming units) for each composition tested was calculated. A lower Log10 CFU/ml indicates that the dentifrice tested has greater efficacy in inhibiting biofilm growth.

The results of the tests are shown in Examples 1 and 2, below.

### Example 1

In the first round of biofilm reduction tests, the formulations listed in Table 1 were evaluated for their ability to reduce biofilm growth.

**Table 1**

| | **Composition 1** | **Composition 2** | **Composition 3** | **Composition 4** |
|---|---|---|---|---|
| Zinc oxide | 1.00 | 1.00 | 1.00 | 0 |
| Zinc citrate trihydrate | 0.50 | 0.50 | 0.50 | 0 |
| L-arginine | 1.00 | 0 | 0 | 0 |
| L-serine | 0.10 | 0.15 | 0 | 0 |
| Surfactants | 3.25 | 3.25 | 3.25 | 3.25 |
| Fluoride sources | 1.10 | 1.10 | 1.10 | 1.10 |
| Anticalculus agents | 0.50 | 0.50 | 0.50 | 0.50 |
| Abrasives (silica) | 15.00 | 15.00 | 15.00 | 15.00 |
| Orally acceptable vehicle | 75.45 | 76.40 | 76.55 | 78.05 |
| Minors | 2.10 | 2.10 | 2.10 | 2.10 |
| **Total** | 100.00 | 100.00 | 100.00 | 100.00 |

Composition 1 (containing 1 weight % ZnO, 0.5 weight % ZnCit, 1 weight % arginine, 0.1 weight % serine, and an abrasive) was a composition of the present invention. The biofilm reduction efficacy of Composition 1 was compared with that of a control (Composition 3) which contained 1 weight % ZnO, 0.5 weight % ZnCit, and an abrasive, but no arginine or serine, and also with that of a placebo (Composition 4) which contained an abrasive, but no zinc compounds, no arginine and no serine.

The biofilm reduction efficacy of Composition 1 was also compared with that of Composition 2, which contained 1 weight % ZnO, 0.5 weight % ZnCit, 0.15 weight % serine, and an abrasive, but no arginine. Composition 2 had been previously shown to exhibit the greatest biofilm reduction efficacy of a range of ZnO/ZnCit/serine-containing compositions having different concentrations of serine (i.e. 0.15 weight % serine was previously found to be the optimum concentration of serine for reduction of biofilm by compositions containing ZnO, ZnCit and serine).

The abrasive used was the same in each of Compositions 1 to 4.

The results obtained using the biofilm growth inhibition test as described above are shown in Table 1, with the average log10 CFU/ml obtained from the disk incubated for 8 days in step 6 of the method. The statistical significance of the results was evaluated by Analysis of Variance for the factor "log CFU/ml" using General Linear Model in Minitab. In Table 2 below, formulae where the avg. log10 CFU/ml results share the same superscript letter do not show a significant difference in their ability to inhibit biofilm growth. The significance level used was 5% (also expressed as 95% confidence level).

**Table 2**

| No. | Formula | Avg. log10 CFU/ml |
|---|---|---|
| 1 | 1wt% ZnO, 0.5wt% ZnCit, 1wt% arginine, 0.1wt% serine, abrasive | 4.06^{CD} |
| 2 | 1wt% ZnO, 0.5wt% ZnCit, 0.15wt% serine, abrasive | 4.24^{BC} |
| 3 | 1wt% ZnO, 0.5wt% ZnCit, abrasive | 4.44^{B} |
| 4 | Placebo (abrasive, but no zinc and no amino acids) | 6.28^{A} |

As shown in Table 2, Composition 1 (of the present invention) exhibited greater biofilm reduction efficacy than Compositions 2, 3 and 4. In particular, Composition 1 showed greater biofilm reduction efficacy than the composition containing 0.15 weight % serine (Composition 2), which had previously been shown to be the most efficacious ZnO/ZnCit/serine-containing composition in terms of biofilm reduction efficacy, as discussed above.

### Example 2

In the second round of biofilm reduction tests, the formulations listed in Table 3 were evaluated for their ability to reduce biofilm growth.

**Table 3**

| | **Composition 5** | **Composition 6** | **Composition 7** | **Composition 8** |
|---|---|---|---|---|
| Zinc oxide | 1.00 | 1.00 | 0 | 1.00 |
| Zinc citrate trihydrate | 0.50 | 0.50 | 0 | 0.50 |
| L-arginine | 1.00 | 1.50 | 0 | 0 |
| L-serine | 0.10 | 0 | 0 | 0 |
| Surfactants | 3.25 | 3.25 | 1.50 | 3.25 |
| Fluoride sources | 1.10 | 0.32 | 0.24 | 0.32 |
| Anticalculus agents | 0.50 | 0.50 | 0 | 0.50 |
| Abrasives (silica) | 15.00 | 15.00 | 18.50 | 15.00 |
| Orally acceptable vehicle | 75.45 | 75.83 | 77.41 | 77.33 |
| Antibacterial agent (triclosan) | 0 | 0 | 0.30 | 0 |
| Minors | 2.10 | 2.10 | 2.05 | 2.10 |
| **Total** | 100.00 | 100.00 | 100.00 | 100.00 |

Composition 5 (containing 1 weight % ZnO, 0.5 weight % ZnCit, 1 weight % arginine, 0.1 weight % serine, and an abrasive) was a composition of the present invention. The biofilm reduction efficacy of Composition 5 was compared with that of a control (Composition 8) which contained 1 weight % ZnO, 0.5 weight % ZnCit, and an abrasive, but no arginine or serine, and also with Composition 7, which contained 0.3 weight % triclosan and an abrasive, but no zinc compounds and no amino acids.

The biofilm reduction efficacy of Composition 5 was also compared with that of Composition 6, which contained 1 weight % ZnO, 0.5 weight % ZnCit, 1.5 weight % arginine, and an abrasive, but no serine. Composition 6 had been previously shown to exhibit the greatest biofilm reduction efficacy of a range of ZnO/ZnCit/arginine-containing compositions having different concentrations of arginine (i.e. 1.5 weight % arginine was previously found to be the optimum concentration of arginine for reduction of biofilm by compositions containing ZnO, ZnCit and arginine).

The abrasive used was the same in each of Compositions 5 to 8, and was the same abrasive as used in Compositions 1 to 4.

The results obtained using the biofilm growth inhibition test as described above are shown in Table 4, with the average log10 CFU/ml obtained from the disk incubated for 8 days in step 6 of the method. The statistical significance of the results was evaluated by Analysis of Variance for the factor "log CFU/ml" using General Linear Model in Minitab. In Table 4 below, formulae where the avg. log10 CFU/ml results share the same superscript letter do not show a significant difference in their ability to inhibit biofilm growth. The significance level used was 5% (also expressed as 95% confidence level).

**Table 4**

| No. | Formula | Avg. log10 CFU/ml |
|---|---|---|
| 5 | 1wt% ZnO, 0.5wt% ZnCit, 1wt% arginine, 0.1wt% serine, abrasive | 4.10^{B} |
| 6 | 1wt% ZnO, 0.5wt% ZnCit, 1.5wt% arginine, abrasive | 4.29^{AB} |
| 7 | Control (containing 0.3 weight % triclosan and an abrasive, but no zinc compounds or amino acids) | 4.31^{AB} |
| 8 | 1wt% ZnO, 0.5wt% ZnCit, abrasive | 4.49^{A} |

As shown in Table 4, Composition 5 (of the present invention) exhibited greater biofilm reduction efficacy than Compositions 6, 7 and 8. In particular, Composition 5 showed greater biofilm reduction efficacy than the composition containing 1.5 weight % arginine (Composition 6), which had previously been shown to be the most efficacious ZnO/ZnCit/arginine-containing composition in terms of biofilm reduction efficacy, as discussed above.

It can be seen from the above results in Examples 1 and 2 that the compositions of the present invention (containing both arginine and serine and a combination of zinc oxide and zinc citrate) provided increased biofilm reduction efficacy as compared to the optimized comparative formulas which contained either serine only or arginine only, despite the concentrations of serine and arginine in Compositions 1 and 5, above, being below the optimized levels as present in comparative Compositions 2 and 6. The compositions of the present invention therefore provide improved biofilm reduction efficacy. Further, this improved biofilm reduction efficacy is maintained even when the concentrations of serine and arginine are reduced to levels below those previously found to be optimal.

## Claims

1. An oral care composition comprising:
a. arginine, in free or salt form;
b. serine
c. zinc oxide; and
d. zinc citrate.

2. The oral care composition of claim 1, wherein the total concentration of zinc oxide and zinc citrate in the composition is from 0.2 weight % to 5 weight %, based on the total weight of the composition.

3. The oral care composition of any preceding claim, wherein the weight ratio of zinc oxide to zinc citrate is from 1.5:1 to 4.5:1, preferably wherein the weight ratio of zinc oxide to zinc citrate is from 1.5:1 to 4:1, from 1.7:1 to 2.3:1, from 1.9:1 to 2.1:1, or about 2:1.

4. The oral care composition of any preceding claim, wherein the composition comprises zinc oxide in an amount of from 0.5 weight % to 1.5 weight % and zinc citrate in an amount of from 0.25 weight % to 0.75 weight %, based on the total weight of the composition.

5. The oral care composition of claim 4, wherein the composition comprises zinc oxide in an amount of about 1 weight % and zinc citrate in an amount of about 0.5 weight %, based on the total weight of the composition.

6. The oral care composition of any preceding claim, wherein the arginine is present, in free or salt form, in an amount of from 0.5 weight % to 8 weight %, and the serine is present in an amount of from 0.01 weight % to 0.8 weight %, based on the total weight of the composition, optionally wherein the arginine is present in an amount of from 0.5 weight % to 1.5 weight % and the serine is present in an amount of from 0.05 weight % to 0.2 weight %, based on the total weight of the composition.

7. The oral care composition of any preceding claim, wherein the weight ratio of serine to arginine is from 1:5 to 1:15, from 1:7 to 1:12, or about 1:10.

8. The oral care composition of any preceding claim, wherein the arginine is L-arginine and/or wherein the serine is L-serine.

9. The oral care composition of any preceding claim, wherein the arginine is present as free arginine or wherein the arginine is present as arginine hydrochloride, arginine bicarbonate, or arginine phosphate.

10. The oral care composition of any preceding claim, wherein the weight ratio of the zinc oxide and zinc citrate to the arginine and serine in the composition is from 2:1 to 1:1, optionally wherein the weight ratio of the zinc oxide and zinc citrate to the arginine and serine in the composition is about 1.4:1.

11. The oral care composition of any preceding claim, further comprising an abrasive, optionally
wherein the abrasive is selected from a silica abrasive, aluminum oxide, aluminum silicate, calcined alumina, bentonite, insoluble phosphate, natural calcium carbonate, precipitated calcium carbonate, and mixtures thereof, preferably
wherein the abrasive is a silica abrasive, or
wherein the abrasive is natural calcium carbonate or precipitated calcium carbonate.

12. The oral care composition of any preceding claim, wherein the oral care composition is a dentifrice, a toothpaste, a gel, a tooth powder, a mouthwash, a mouthrinse, a lozenge, a tablet, a spray, a gum, or a film.

13. The oral care composition according to any one of the preceding claims for use in a method of reducing or inhibiting biofilm formation in an oral cavity.

14. An oral care composition according to any one of the preceding claims for use in a method of reducing or inhibiting biofilm formation in an oral cavity, the method comprising: contacting the oral cavity with the oral care composition.

## Patentansprüche

1. Mundpflegezusammensetzung, umfassend:
a. Arginin, in freier Form oder Salzform;
b. Serin
c. Zinkoxid; und
d. Zinkcitrat.

2. Mundpflegezusammensetzung nach Anspruch 1, wobei die Gesamtkonzentration an Zinkoxid und Zinkcitrat in der Zusammensetzung von 0,2 Gew.-% bis 5 Gew.-% ist, bezogen auf das Gesamtgewicht der Zusammensetzung.

3. Mundpflegezusammensetzung nach irgendeinem vorhergehenden Anspruch, wobei das Gewichtsverhältnis Zinkoxid zu Zinkcitrat von 1,5:1 bis 4,5:1 ist, vorzugsweise ist das Gewichtsverhältnis Zinkoxid zu Zinkcitrat von 1,5:1 bis 4:1, von 1,7:1 bis 2,3:1, von 1,9:1 bis 2,1:1 oder etwa 2:1.

4. Mundpflegezusammensetzung nach irgendeinem vorhergehenden Anspruch, wobei die Zusammensetzung Zinkoxid in einer Menge von 0,5 Gew.-% bis 1,5 Gew.-% und Zinkcitrat in einer Menge von 0,25 Gew.-% bis 0,75 Gew.-% umfasst, bezogen auf das Gesamtgewicht der Zusammensetzung.

5. Mundpflegezusammensetzung nach Anspruch 4, wobei die Zusammensetzung Zinkoxid in einer Menge von etwa 1 Gew.-% und Zinkcitrat in einer Menge von etwa 0,5 Gew.-% umfasst, bezogen auf das Gesamtgewicht der Zusammensetzung.

6. Mundpflegezusammensetzung nach irgendeinem vorhergehenden Anspruch, wobei Arginin in freier Form oder Salzform vorliegt, in einer Menge von 0,5 Gew.-% bis 8 Gew.-%, und Serin liegt vor eine einer Menge von 0,01 Gew.-% bis 0,8 Gew.-%, bezogen auf das Gesamtgewicht der Zusammensetzung, gegebenenfalls ist das Arginin in einer Menge von 0,5 Gew.-% bis 1,5 Gew.-% vorhanden und das Serin ist in einer Menge von 0,05 Gew.-% bis 0,2 Gew.-% vorhanden, bezogen auf das Gesamtgewicht der Zusammensetzung.

7. Mundpflegezusammensetzung nach irgendeinem vorhergehenden Anspruch, wobei das Gewichtsverhältnis Serin zu Arginin von 1:5 bis 1:15, von 1:7 bis 1:12 oder etwa 1:10 ist.

8. Mundpflegezusammensetzung nach irgendeinem vorhergehenden Anspruch, wobei das Arginin L-Arginin ist, und/oder wobei das Serin L-Serin ist.

9. Mundpflegezusammensetzung nach irgendeinem vorhergehenden Anspruch, wobei das Arginin als freies Arginin vorliegt, oder wobei das Arginin als Argininhydrochlorid, Argininbicarbonat oder Argininphosphat vorliegt.

10. Mundpflegezusammensetzung nach irgendeinem vorhergehenden Anspruch, wobei das Gewichtsverhältnis Zinkoxid und Zinkcitrat zu Arginin und Serin in der Zusammensetzung von 2:1 bis 1:1 ist, gegebenenfalls ist das Gewichtsverhältnis Zinkoxid und Zinkcitrat zu Arginin und Serin in der Zusammensetzung etwa 1,4:1.

11. Mundpflegezusammensetzung nach irgendeinem vorhergehenden Anspruch, weiterhin umfassend ein Abrasivstoff, gegebenenfalls ist der Abrasivstoff ausgewählt aus Silicaabrasiv, Aluminiumoxid, Aluminiumsilicat, kalziniertem Alumina, Bentonit, unlöslichem Phosphat, natürlichem Calciumcarbonat, ausgefälltem Calciumcarbonat und Mischungen davon, vorzugsweise ist der Abrasivstoff ein Silicaabrasiv, oder der Abrasivstoff ist natürliches Calciumcarbonat oder ausgefälltes Calciumcarbonat.

12. Mundpflegezusammensetzung nach irgendeinem vorhergehenden Anspruch, wobei die Mundpflegezusammensetzung ein Zahnputzmittel, eine Zahnpasta, ein Gel, ein Zahnpulver, eine Mundwäsche, eine Mundspülung, ein Bonbon, eine Tablette, ein Spray, ein Gummi oder ein Film ist.

13. Mundpflegezusammensetzung nach irgendeinem der vorhergehenden Ansprüche zu Verwendung in einem Verfahren zur Verminderung oder Inhibierung von Biofilmbildung in einer Mundhöhle.

14. Mundpflegezusammensetzung nach irgendeinem der vorhergehenden Ansprüche zur Verwendung in einem Verfahren zur Verminderung oder Inhibierung von Biofilmbildung in einer Mundhöhle, wobei das Verfahren umfasst:
In-Kontaktbringen der Mundhöhle mit der Mundpflegezusammensetzung.

## Revendications

1. Composition de soin buccal comprenant :
a. de l'arginine, sous forme libre ou de sel ;
b. de la sérine ;
c. de l'oxyde de zinc ; et
d. du citrate de zinc.

2. Composition de soin buccal selon la revendication 1, dans laquelle la concentration totale d'oxyde de zinc et de citrate de zinc dans la composition est de 0,2 % en poids à 5 % en poids, sur la base du poids total de la composition.

3. Composition de soin buccal selon l'une quelconque des revendications précédentes, dans laquelle le rapport en poids de l'oxyde zinc au citrate de zinc est de 1,5:1 à 4,5:1, de préférence dans laquelle le rapport en poids de l'oxyde de zinc au citrate de zinc est de 1,5:1 à 4:1, de 1,7:1 à 2,3:1, de 1,9:1 à 2,1:1, ou d'environ 2:1.

4. Composition de soin buccal selon l'une quelconque des revendications précédentes, dans laquelle la composition comprend de l'oxyde de zinc dans une quantité de 0,5 % en poids à 1,5 % en poids et du citrate de zinc dans une quantité de 0,25 % en poids à 0,75 % en poids, sur la base du poids total de la composition.

5. Composition de soin buccal selon la revendication 4, dans laquelle la composition comprend de l'oxyde de zinc dans une quantité d'environ 1 % en poids et du citrate de zinc dans une quantité d'environ 0,5 % en poids, sur la base du poids total de la composition.

6. Composition de soin buccal selon l'une quelconque des revendications précédentes, dans laquelle l'arginine est présente, sous forme libre ou de sel, dans une quantité de 0,5 % en poids à 8 % en poids et la sérine est présente dans une quantité de 0,01 % en poids à 0,8 % en poids, sur la base du poids total de la composition, facultativement dans laquelle l'arginine est présente dans une quantité de 0,5 % en poids à 1,5 % en poids et la sérine est présente dans une quantité de 0,05 % en poids à 0,2 % en poids, sur la base du poids total de la composition.

7. Composition de soin buccal selon l'une quelconque des revendications précédentes, dans laquelle le rapport en poids de la sérine sur l'arginine est de 1:5 à 1:15, de 1:7 à 1:12 ou d'environ 1:10.

8. Composition de soin buccal selon l'une quelconque des revendications précédentes, dans laquelle l'arginine est la L-arginine et/ou dans laquelle la sérine est la L-sérine.

9. Composition de soin buccal selon l'une quelconque des revendications précédentes, dans laquelle l'arginine est présente en tant qu'arginine libre ou dans laquelle l'arginine est présente en tant que chlorhydrate d'arginine, bicarbonate d'arginine ou phosphate d'arginine.

10. Composition de soin buccal selon l'une quelconque des revendications précédentes, dans laquelle le rapport en poids de l'oxyde de zinc et du citrate de zinc à l'arginine et la sérine dans la composition est de 2:1 à 1:1, facultativement dans laquelle le rapport en poids de l'oxyde de zinc et du citrate de zinc à l'arginine et la sérine dans la composition est d'environ 1,4:1.

11. Composition de soin buccal selon l'une quelconque des revendications précédentes, comprenant en outre un abrasif, facultativement
dans laquelle l'abrasif est choisi parmi une silice abrasive, l'oxyde d'aluminium, le silicate d'aluminium, l'alumine calcinée, la bentonite, le phosphate insoluble, le carbonate de calcium naturel, le carbonate de calcium précipité et leurs mélanges, de préférence
dans laquelle l'abrasif est une silice abrasive, ou
dans laquelle l'abrasif est le carbonate de calcium naturel ou le carbonate de calcium précipité.

12. Composition de soin buccal selon l'une quelconque des revendications précédentes, dans laquelle la composition est un dentifrice, une pâte dentifrice, un gel, une poudre dentifrice, une eau dentifrice, un bain de bouche, une pastille, un comprimé, une pulvérisation, une gomme ou un film.

13. Composition de soin buccal selon l'une quelconque des revendications précédentes pour une utilisation dans un procédé de réduction ou d'inhibition d'une formation de biofilm dans une cavité buccale.

14. Composition de soin buccal selon l'une quelconque des revendications précédentes pour une utilisation dans un procédé de réduction ou d'inhibition d'une formation de biofilm dans une cavité buccale, le procédé comprenant : mettre en contact la cavité buccale avec la composition de soin buccal.
